# EUROPÄISCHE PATENTSCHRIFT

(11) **EP 3 238 667 B1**
(45) Veröffentlichungstag und Bekanntmachung des Hinweises auf die Patenterteilung: **10.10.2018**
(21) Anmeldenummer: 16166902.3
(22) Anmeldetag: 25.04.2016
(51) Int. Cl.: A61F 2/78

(54) **LINER ZUM ÜBERZIEHEN ÜBER EINEN GLIEDMASSENSTUMPF**
LINER FOR COATING OVER A LIMB STUMP
ENVELOPPE DESTINEE A RECOUVRIR UN MOIGNON

(43) Veröffentlichungstag der Anmeldung: 01.11.2017
(73) Patentinhaber: Össur Iceland EHF, 110 Reykjavik (IS)
(72) Erfinder: KURTH, Christof, 95500 Heinersreuth (DE)
(74) Vertreter: Klunker IP Patentanwälte PartG mbB

(56) Entgegenhaltungen:
- EP-A1- 2 745 807
- WO-A2-97/34548
- US-A1- 2013 331 952

## Beschreibung

Die Erfindung betrifft einen Liner zum Überziehen über einen Gliedmaßenstumpf zur Fixierung eines Prothesenschafts, mit einem hohlen Linerkörper und wenigstens einer umlaufenden, seitlich vom Linerkörper abstehenden flexiblen Dichtlippe zur dichten Anlage an der Innenseite des Prothesenschafts.

Ein solcher Liner dient dazu, eine hinreichend feste Fixierung eines Prothesenschafts an einem Gliedmaßenstumpf zu ermöglichen. Der Liner besteht aus einem hinreichend flexiblen Material, insbesondere einem Silikon, so dass er sich gut der Form des Gliedmaßenstumpfes, über den er gezogen wird, anpassen kann. Im Bereich des distalen Endes des hohlen Linerkörpers ist wenigstens eine, zumeist mehrere axial voneinander beabstandete Dichtlippen ebenfalls aus einem flexiblen Material, vornehmlich dem gleichen Material wie dem des Linerkörpers, also einem Silikon, vorgesehen. Diese Dichtlippe oder diese mehreren Dichtlippen legen sich an die Innenseite des Prothesenschafts an, wenn der mit dem Liner versehene Stumpf in den Prothesenschaft eingeführt wird. Durch das Einführen wird Luft aus dem Prothesenschaft herausgedrückt, wozu üblicherweise ein entsprechendes Ventil vorgesehen ist, das bevorzugt so im Prothesenschaft angeordnet ist, dass es sich bei arretiertem Liner distal der distalsten Dichtlippe befindet. In dieser arretierten Endstellung ist der Liner über die eine oder die mehreren Dichtlippen zum Prothesenschaft hin abgedichtet, so dass, wenn der Stumpf nebst Liner aus dem Prothesenschaft herausgezogen werden würde, ein Unterdruck im Inneren des Prothesenschafts erzeugt wird, der dieses Herausziehen verhindert. Die Funktion eines solchen Liners ist hinreichend bekannt.

Es liegt auf der Hand, dass die Geometrie verschiedener Gliedmaßenstümpfe hochgradig unterschiedlich ist. Dies ist insbesondere im Unterschenkelbereich der Fall. Einerseits variiert der Umfang mitunter beachtlich, andererseits ergeben sich auch ausgeprägte Geometrieunterschiede im Bereich der Tibiakante, also der Schienbeinkante. Die Schienbeinkante ist bei kräftigen, dickeren Unterschenkeln weit weniger ausgeprägt als bei schlanken, dünnen Unterschenkeln, wo die Tibiakante teilweise sehr dominant den vorderen Unterschenkelbereich definiert. Die Geometrie der Querschnittsflächen weicht hier von einer üblicherweise rundlichen Geometrie ab, es ergibt sich eine eher kantigere, von der Tibiakante definierte Geometrie, die medial zur Tibikante eine Abflachung aufweist. Es hat sich nun gezeigt, dass es mitunter bei Trägern solcher Liner, deren Unterschenkel solche eine von einer runden Querschnittsfläche starke abweichende Geometrie aufweist, zu einem Ablösen der einen oder der mehreren Dichtlippen von der Innenseite des Prothesenschaftes kommt, wodurch der Unterdruck im Prothesenschaft verloren geht und der Prothesenschaft nicht mehr zuverlässig mit dem Liner verbunden ist. Dieses Ablösen ist auf die von der Ringform des Linerkörpers respektive der einen oder mehreren Dichtlippen teilweise deutlich abweichenden Geometrie des Unterschenkels im Bereich der Tibiakante zurückzuführen.

Insbesondere beim Sitzen, wenn der Stumpf im Prothesenschaft quasi nach hinten kippt, wurde festgestellt, dass es in manchen Fällen zu einem Ablösen der einen oder mehreren Dichtlippen im vorderen Linerbereich kommt, so dass auch in solchen Situationen der Unterdruck im Prothesenschaft verloren gehen kann. Dieser Effekt kann durch eine eher kantige Geometrie im Tibiakantenbereich noch verstärkt werden.

Der Erfindung liegt damit das Problem zugrunde, einen Liner anzugeben, der demgegenüber verbessert ist.

Zur Lösung dieses Problems ist bei einem Liner der eingangs genannten Art erfindungsgemäß vorgesehen, dass die Höhe der Dichtlippe um ihren Umfang von einem ersten Höhenwert an eine erste Umfangsposition oder in einem ersten Umfangsbereich zu einem zweiten Höhenwert an einer zweiten Umfangsposition oder in einem zweiten Umfangsbereich variiert.

Anders als im bisherigen Stand der Technik, bei dem wenigstens eine ringförmig umlaufende Dichtlippe um den gesamten Umfang stets eine gleichbleibende, konstante Höhe aufweist, ist erfindungsgemäß eine definierte Variation der Dichtlippenhöhe vorgesehen. Die Dichtlippenhöhe variiert zwischen einem ersten und einem zweiten, kleineren Höhenwert, wobei die beiden Höhenwerte jeweils an definierten ersten und zweiten Umfangspositionen gegeben sein können, oder in entsprechenden ersten und zweiten Umfangsbereichen. Im erst genannten Fall ist also eine Umfangsposition mit dem ersten Höhenwert und eine Umfangsposition mit dem zweiten Höhenwert vorgesehen, während im zweitgenannten Fall jeweils Umfangsbereiche vorgesehen sind, in denen ein konstanter erster Höhenwert und ein konstanter zweiter Höhenwert gegeben ist. Selbstverständlich sind auch Mischformen denkbar, das heißt, dass an einer Position ein definierter erster Höhenwert gegeben ist, von dem ausgehend die Dichtlippe in einem Umfangsbereich, in dem ein konstanter zweiter Höhenwert gegeben ist, abnimmt und ähnliches.

Diese Höhenvariation ermöglicht es nun, die Dichtlippenhöhe so anzupassen, dass die Höhe in einem Umfangsbereich, der in der Tragstellung vorne, also im Bereich der Tibiakante positioniert ist, größer ist als in anderen Bereichen, beispielsweise an der gegenüberliegenden Linerseite. Das heißt, dass in einem bezogen auf die Tragstellung kritischen Linerbereich, wo es also zu einem Ablösen der Dichtlippe kommen kann, die Dichtlippe länger ist, so dass, selbst wenn der Stumpf im Prothesenschaft geringfügig verkippt, aufgrund der größeren Dichtlippenlänge immer noch eine dichte Anlage der Dichtlippe zum Prothesenschaft gegeben ist. Durch diese Höhenvariation um den Dichtlippenumfang wird folglich einer durch die Bewegung oder Sitzposition hervorgerufenen Verlagerung der Dichtlippe dahingehend Rechnung getragen, dass gerade in dem oder den Bereichen, wo eine Ablösung der Dichtlippe unter Verlust des Unterdrucks möglich sein kann, die Dichtlippe länger ausgeführt ist, mithin also eine dichte Anlage am Prothesenschaft auch bei einer größeren Verlagerung der Dichtlippe relativ zum Linerkörper sichergestellt ist. Durch den erfindungsgemäßen Liner ist sichergestellt, dass sich die Dichtlippe auch in unterschiedlichen Bewegungs- oder Sitzpositionen etc. nicht vom Prothesenschaft lösen kann, so dass der Unterdruck auch in diesen Situationen aufrechterhalten werden kann, auch wenn es zu einer Verlagerung des Stumpfes im Prothesenschaft kommt, respektive die Stumpfgeometrie eine ausgeprägte Tibiakante mit starker Abweichung von einer eher rundlichen Umfangsform aufweist.

Dabei ist die erste Umfangsposition oder der erste Umfangsbereich bevorzugt gegenüberliegend zu der zweiten Umfangsposition oder dem zweiten Umfangsbereich angeordnet. Das heißt, dass die erste Umfangsposition oder der erste Umfangsbereich mit dem ersten Höhenwert der Dichtlippenhöhe frontal ausgebildet respektive vorgesehen ist, während die zweite Umfangsposition oder der zweite Umfangsbereich mit dem zweiten Höhenwert dorsal, also gegenüberliegend und quasi um 180° versetzt, vorgesehen ist. Dies ist zweckmäßig, als üblicherweise im dorsalen Bereich der Unterschenkelstumpf eine rundliche Form aufweist, die zwangsläufig nicht zu einem unbeabsichtigten, geometriebedingten Ablösen führt.

Um ein Ablösen der Dichtlippe auch im Bereich der Abflachung, welche medial zur Tibikante verläuft, zu verhindern, ist der Bereich der größten Dichtlippenhöhe alternativ in dem abgeflachten Bereich des Unterschenkels vorgesehen.

Bevorzugt sind am Liner mehrere axial geringfügig voneinander beabstandete Dichtlippen vorgesehen, wobei die Höhe nur einer Dichtlippe, mehrerer Dichtlippen oder aller Dichtlippen in der erfindungsgemäßen Weise variieren kann. Dabei können die ersten und zweiten Höhenwerte der unterschiedliche Höhen aufweisenden Dichtlippen gleich sein, sie können sich aber auch von Dichtlippe zu Dichtlippe unterscheiden.

Hinsichtlich des konkreten Dichtlippendesigns respektive Höhenverlaufs sind unterschiedliche erfindungsgemäße Ausgestaltungen denkbar. Nach einer ersten Erfindungsalternative kann die Höhe ausgehend von einer den ersten Höhenwert aufweisenden ersten Umfangsposition, bevorzugt kontinuierlich oder stufenweise, zu dem an einer zweiten Umfangsposition gegebenen zweiten Höhenwert hin abnehmen. Es sind also zwei ausgezeichnete Umfangspositionen vorgesehen, die, wie bereits beschrieben, bevorzugt einander gegenüberliegend frontal und dorsal ausgebildet sind. Der erste Höhenwert stellt dabei einen Maximalwert und der zweite Höhenwert einen Minimalwert dar. Die Dichtlippenhöhe nimmt kontinuierlich vom Maximalwert um den Umfang zum Minimalwert hin ab.

Gemäß einer Erfindungsalternative kann die Höhe in einem Umfangsbereich gleichbleibend den ersten Höhenwertwert aufweisen und anschließend, bevorzugt kontinuierlich oder stufenweise, zu dem zweiten Höhenwert hin abnehmen. Hier ist also frontal ein Maximalwert-Umfangsbereich mit konstanter Dichtlippenhöhe vorgesehen, wobei die Dichtlippenhöhe beidseits dieses Umfangsbereichs zu einer Umfangsposition, die den zweiten Höhenwert, auch hier ein Minimalwert aufweist, vorzugsweise kontinuierlich hin abnimmt. Es ist also ein Maximalwertbereich und eine Minimalwertposition vorgesehen.

Alternativ kann die Ausgestaltung auch umgekehrt sein, nämlich dass die Höhe ausgehend von einer den ersten Höhenwert, auch hier ein Maximalwert, aufweisenden Umfangsposition, bevorzugt kontinuierlich oder stufenweise, zu dem zweiten Höhenwert, hier wieder ein Minimalwert, hin abnimmt, der in einem Umfangsbereich gleichbleibend gegeben ist. Hier ist also eine Maximalwertposition und ein Minimalwertbereich vorgesehen. Wieder nimmt die Dichtlippenhöhe von der Maximalwertposition beidseits vorzugsweise kontinuierlich ab, bis der Minimalwert erreicht ist, der im Umfangsbereich gegeben ist.

Eine weitere Erfindungsalternative sieht vor, dass die Höhe in einem ersten Umfangsbereich gleichbleibend den ersten Höhenwert, der wieder einen Maximalwert darstellt, aufweist, dass die Höhe in einem daran anschließenden Übergangsbereich, bevorzugt kontinuierlich oder stufenweise, abnimmt, und dass die Höhe in einem daran anschließenden zweiten Umfangsbereich gleichbleibend den zweiten Höhenwert, der wieder einen Minimalwert definiert, aufweist. Hier ist also ein Maximalwertbereich und ein Minimalwertbereich gegeben. Zwischen den beiden Bereichen ist jeweils beidseits am Liner jeweils ein Übergangsbereich gegeben, innerhalb dem die Höhe bevorzugt kontinuierlich vom Maximalwertbereich zum Minimalwertbereich hin abnimmt.

In diesem Zusammenhang ist es denkbar, dass die Höhe in einem oder in beiden Übergangsbereichen abschnittsweise kleiner ist als der im zweiten Umfangsbereich gegebene zweite Höhenwert, oder größer als der im ersten Umfangsbereich gegebene erste Höhenwert. Gemäß dieser Erfindungsausgestaltung ist quasi im lateralen Bereich oder in beiden lateralen Bereichen, also in den Abschnitten zwischen den frontalen und dorsalen Bereichen, eine gezielte Höhenreduzierung unter den zweiten Höhenwert (der in diesem Fall keinen Minimalwert darstellt) Minimalwert vorgesehen, also in anatomisch sinnvollen Bereichen. Alternativ kann lokal gesehen in diesen Bereichen auch eine geringfügige Höhenerhöhung über den ersten Höhenwert (der in diesem Fall keinen Maximalwert darstellt) hinaus erfolgen, wenn dies anatomisch zweckmäßig ist.

Der erste Höhenwert der Dichtlippenhöhe, der wie beschrieben insbesondere im frontalen Bereich gegeben ist, sollte zwischen 4 - 8 mm, insbesondere bei 6 mm liegen, während der zweite Höhenwert, der bevorzugt gegenüberliegend im dorsalen Bereich gegeben ist, zwischen 2 - 5 mm, insbesondere bei 4 mm liegt. Die beiden Höhenwerte können beliebig gewählt werden, solange der frontale erste Höhenwert an der ersten Umfangsposition oder im ersten Umfangsbereich größer ist als der dorsale zweite Höhenwert an der zweiten Umfangsposition oder im zweiten Umfangsbereich.

Die Dichtlippe weist gemäß einer besonders zweckmäßigen Weiterbildung der Erfindung bevorzugt eine trapezförmige Querschnittsform auf, mit zwei ebenen Seitenflächen und einer diese verbindenden ebenen Außenfläche. Eine solche Trapezform ist insbesondere in Bezug auf die Herstellung zweckmäßig, da sich eine solche Kontur auf einfache Weise in das Gießwerkzeug einbringen lässt, beispielsweise unter Verwendung eines Formfräsers oder einer Erodierelektrode. Dabei ist der Winkel, in dem die Dichtlippe an ihrem Fuß in den Linienkörper trifft, unabhängig von der Eintauchtiefe des Ausformungswerkzeugs, das die Dichtlippennut in das Gießwerkezug einbringt, was bei gebogenen oder verrundeten Dichtlippenflanken nicht gewährleistet werden kann. Ein Liner mit einer derartigen trapezförmigen Dichtlippengeometrie ist beispielsweise bereits aus WO 97/34548 bekannt.

Dabei kann der Winkel, den die beiden Außenflächen der Dichtlippen zur Oberfläche des Liners einnehmen, gleich oder unterschiedlich sein, er ist jedoch stets jeweils ungleich 90°. Das heißt, dass der Winkel, den die eine Lippenaußenflanke zur Lineroberfläche respektive Linerebene gleich dem Winkel der zweiten Lippenflanke zur Lineroberfläche oder Linerebene ist, die Dichtlippe weist quasi eine symmetrische Geometrie auf. Jedoch kann alternativ auch der eine Winkel größer als der andere sein, so dass die Dichtlippe eine asymmetrische Geometrie aufweist. Keiner der Winkel weist jedoch 90° auf.

In Weiterbildung der Erfindung kann vorgesehen sein, dass die Wandstärke des Linerkörpers im Bereich der einen oder der mehreren Dichtlippen um den Umfang variiert, wobei die Wandstärke in dem Bereich, in dem die eine oder die mehreren Dichtlippen den ersten Höhenwert aufweisen, größer ist als in dem Bereich, in dem die eine oder die mehreren Dichtlippen den zweiten Höhenwert aufweisen. Bei der Wahl der Wandstärke des Liners ist zu berücksichtigen, dass eine zu hohe Wandstärke im dorsalen Linerkörperbereich, also im Bereich der Kniekehle, vom Träger beim Beugen des Knies aufgrund der zustande kommenden Faltenbildung als unangenehm empfunden werden kann. Andererseits sind die Dichtlippen besonders in Bereichen geringer Weichteildeckung mit ausreichend Silikon zu unterfüttern, damit im Linerkörper an diesen Stellen keine Drucküberhöhung zustande kommt, die das Entstauen des Gliedmaßenstumpfes erschweren oder Läsionen zur Folge haben können. Deshalb weist der Liner zweckmäßigerweise im Linerkörperbereich, in dem die eine oder die mehreren Dichtlippen vorgesehen sind, umlaufend unterschiedliche Wandstärken auf. Die Bereiche, in denen die Wandstärke variiert, sind nicht zwingend mit den Umfangsbereichen, in denen die Höhenvariation vorgesehen ist, identisch.

Bevorzugt ist hierbei die Wandstärke in einer ersten Linerkörperumfangsposition oder in einem ersten Linerkörperumfangsbereich, die oder der der Umfangsposition oder dem Umfangsbereich mit der ersten Dichtlippenhöhe zugeordnet ist, größer als in einer zweiten Linerkörperumfangsposition oder in einem zweiten Linerkörperumfangsbereich, die oder der der Umfangsposition oder dem Umfangsbereich mit der zweiten Dichtlippenhöhe zugeordnet ist. Bevorzugt wird demgemäß insbesondere in dem Bereich der Tibiakante eine hohe Wandstärke des Linerkörpers ausgebildet, um dort eine hinreichende Unterfütterung mit dem weichen, flexiblen Linerkörpermaterial, also dem Silikon, zu erreichen, während im Bereich der Wade eine niedrigere Wandstärke gewählt wird. Ähnlich wie bei den Dichtlippen kann auch die Wandstärke ausgehend von einer Linerkörperumfangsposition zu einer Linerkörperumfangsposition, von einer Linerkörperumfangsposition zu einem Linerkörperumfangsbereich oder umgekehrt, oder von einem Linerkörperumfangsbereich zu einem Linerkörperumfangsbereich variieren.

Dabei kann die Wandstärke in einem Bereich zwischen der ersten und der zweiten Linerkörperumfangsposition oder dem ersten und dem zweiten Linerkörperumfangsbereich kontinuierlich abnehmen. Das heißt, dass sich die Wanddicke von frontal nach dorsal kontinuierlich reduziert. Alternativ kann die Wandstärke in einem Bereich zwischen der ersten und der zweiten Linerkörperumfangsposition oder dem ersten und dem zweiten Linerkörperumfangsbereich ausgehend von der ersten Linerkörperumfangsposition oder vom ersten Linerkörperumfangsbereich zunehmen und zur zweiten Linerumfangsposition oder zum zweiten Linerkörperumfangsbereich hin wieder abnehmen. Das heißt, dass lateral am Linerkörper definierte zusätzliche lokale Aufdickungen, also definierte Wandstärkenvergrößerungen vorgesehen sind, was mitunter aus anatomischen Gründen bzw. einer Anpassung an die Stumpfgeometrie zweckmäßig sein kann. Diese lokale laterale Aufdickung ist insbesondere bei kleineren Linergrößen zweckmäßig. Denn insbesondere bei sehr schlanken Stümpfen wird die Stumpfform weniger durch die Weichteildeckung, sondern durch die darunter liegenden knöchernen Strukturen geprägt. Werden nun gezielt laterale Aufdickungen, also Aufdickungen links und rechts der Tibiakante vorgesehen, so kann eine angenähert kreisförmige Außenkontur des Liners erreicht werden. Alternativ kann auch nur medial der Tibikante aufgedickt werden.

Ist im Bereich der Dichtlippen eine Wandstärkenerhöhung vorgesehen, so ist es zweckmäßig, wenn die Wandstärke des Linerkörpers proximal der einen oder der mehreren Dichtlippen, also des aufgedickten Bereichs, wieder abnimmt, so dass einerseits über dem Knie, also frontal, nicht zu viel Material vorhanden ist, was das Abbeugen des Knies unnötig erschweren würde. Auch im Bereich der Kniekehle ist eine reduzierte Wandstärke vorteilhaft, da so die Faltenbildung beim Abwinkeln minimiert wird.

Aus Stabilitätsgründen ist es zweckmäßig, wenn der Linerkörper in dem Bereich proximal der einen oder der mehreren Dichtlippen mit einem textilen Verstärkungsmaterial versehen ist. Dieses textile Verstärkungsmaterial, beispielsweise ein Textilgewebe oder eine netzartige Textilstruktur oder dergleichen, ermöglicht eine starke Beanspruchung des Liners und reduziert die Gefahr einer Beschädigung desselben, wie beispielsweise eine vom umfänglich beschnittenen proximalen Rand des Liners ausgehende Rißeinleitung insbesondere während des Anziehens und Ausziehens.

Dabei ist es zweckmäßig, wenn das textile Verstärkungsmaterial sowohl in axialer als auch in radialer Richtung dehnbar ist, wobei das Verstärkungsmaterial in beide Richtungen vorzugsweise im gleichen Maße dehnbar ist.

Der Linerkörper sowie die Dichtlippen sind bevorzugt aus einem Silikon.

Weitere Vorteile und Einzelheiten der Erfindung ergeben sich aus den im Folgenden beschriebenen Ausführungsbeispielen sowie anhand der Zeichnung. Dabei zeigen:
- Fig. 1: eine Prinzipdarstellung eines erfindungsgemäßen Liners einer ersten Ausführungsform,
- Fig. 2: eine Längsschnittansicht des Liners aus Fig. 1,
- Fig. 3: eine vergrößerte Teilansicht des Bereichs III aus Fig. 2,
- Fig. 4: eine Querschnittansicht des Liners aus Fig. 1 in der Ebene IV - IV durch eine umlaufende Dichtlippe sowie den Linerkörper einer ersten Ausführungsform,
- Fig. 5: eine Querschnittansicht des Liners aus Fig. 1 in der Ebene V - V durch eine umlaufende Dichtlippe sowie den Linerkörper einer zweiten Ausführungsform,
- Fig. 6: eine Querschnittansicht des Liners aus Fig. 1 in der Ebene VI - VI durch eine umlaufende Dichtlippe sowie den Linerkörper einer dritten Ausführungsform,
- Fig. 7: eine Schnittansicht durch den Linerkörper einer ersten Ausführungsform in der Ebene VII - VII aus Fig. 1,
- Fig. 8: eine Schnittansicht durch den Linerkörper einer zweiten Ausführungsform in der Ebene VIII - VIII aus Fig. 1.

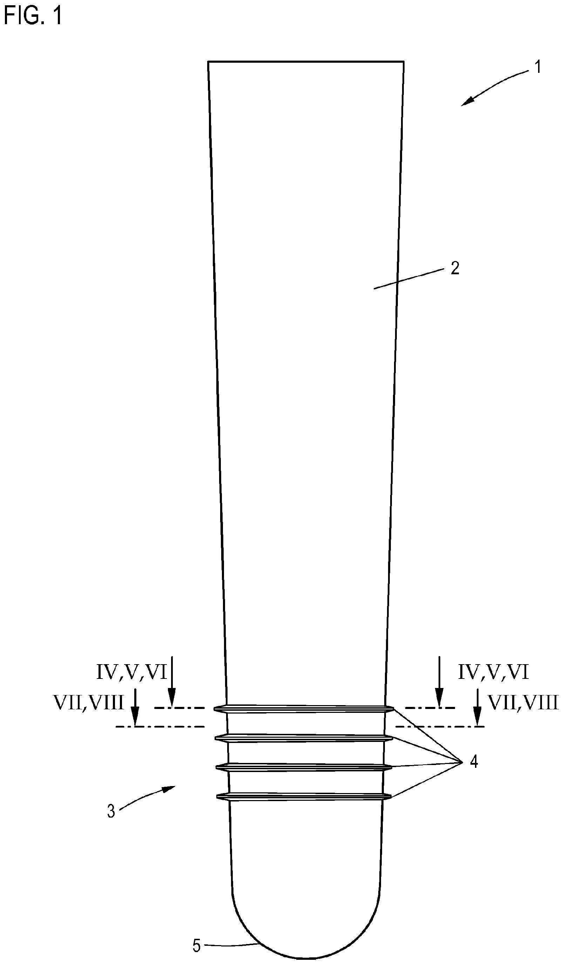

Fig. 1 zeigt einen erfindungsgemäßen Liner 1 einer ersten Ausführungsform zum Überziehen über einen Gliedmaßenstumpf, um einen Prothesenschaft zu fixieren. Der Liner ist insbesondere als Unterschenkelliner ausgelegt, wird also über einen Unterschenkelstumpf gezogen.

Der Liner 1 ist aus einem elastischen Material, vorzugsweise Silikon, gefertigt, so dass er hinreichend flexibel ist und sich der Stumpfgeometrie gut anpassen kann.

Er umfasst einen hohlen Linerkörper 2, der im gezeigten Beispiel eine leicht konische Form aufweist. Im Bereich des distalen Linerkörperendes ist ein Dichtlippenabschnitt 3 vorgesehen umfassend im gezeigten Beispiel vier radial abstehende, ringförmig umlaufende Dichtlippen 4, die axial voneinander beabstandet sind, im gezeigten Beispiel um das gleiche Maß, was jedoch nicht zwingend der Fall sein muss. Unterhalb des Dichtlippenabschnitts 3 geht der Liner 1 in einen endseitigen Kappenabschnitt 5 über, der den Liner nach unten hin abschließt.

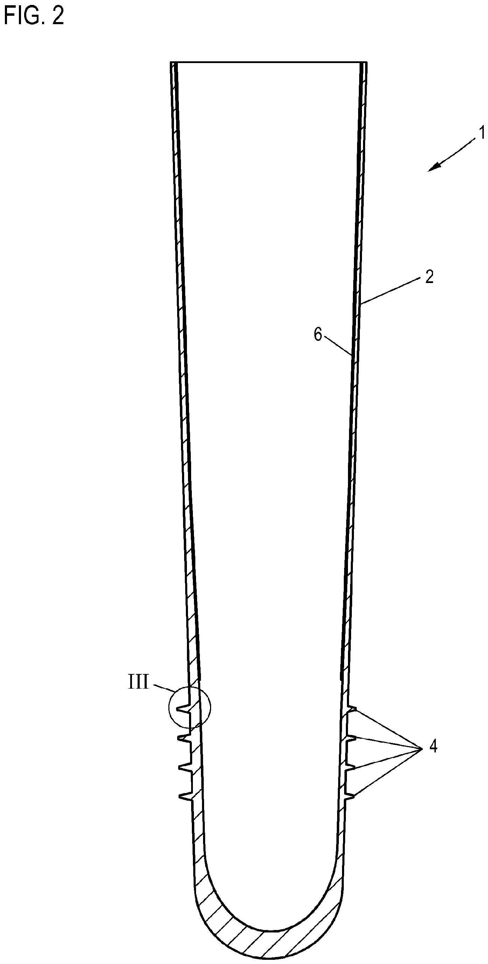

Wie beschrieben besteht der Liner 1 aus einem flexiblen Material wie insbesondere Silikon. Das Material selbst weist üblicherweise bereits eine hinreichende Stabilität respektive Festigkeit auf, so dass der Liner 1 sehr oft angezogen und ausgezogen werden kann, ohne zu verschleißen. Um die Verschleißfestigkeit weiter zu verbessern ist, siehe die Schnittansicht des Liners 1 in Fig. 2, an der Innenseite des Linerkörper 2 ein textiles Verstärkungsmaterial 6 vorgesehen, das zumindest seitens des distalen Randes in die Silikonmatrix eingebunden respektive eingebettet ist, oder an das die Silikonmatrix angebunden ist. Dieses textile Verstärkungsmaterial, beispielsweise ein Gewebe oder Gestrick aus Natur- oder Kunstfaser, weist eine entsprechende Dehnbarkeit auf, die bevorzugt sowohl in axialer als auch in radialer Richtung gegeben ist. Wie Fig. 2 zeigt befindet sich das textile Verstärkungsmaterial ausschließlich oberhalb, also proximal des Dichtlippenabschnitts 3, es endet ein Stück weit beabstandet von der obersten Dichtlippe 4.

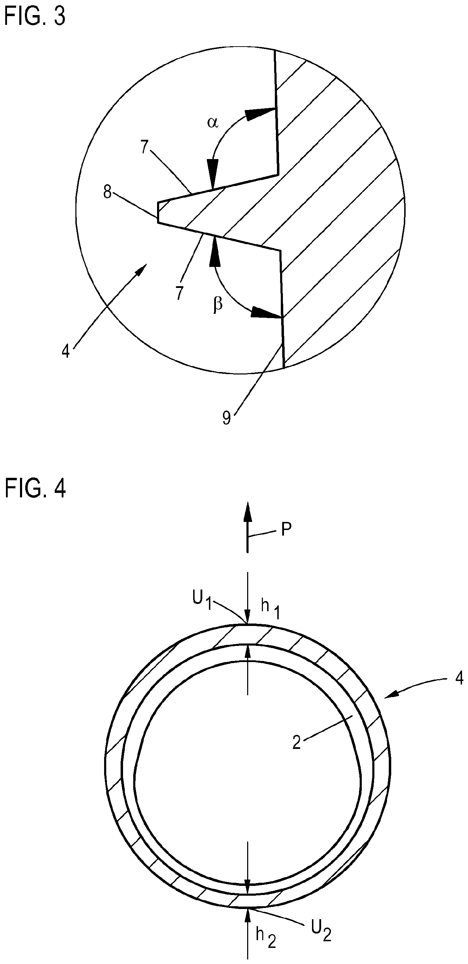

Fig. 3 zeigt eine vergrößerte Teilansicht des Bereichs III aus Fig. 2 unter vergrößerter Darstellung der Geometrie einer Dichtlippe 4. Diese Geometrie ist bei allen vier Dichtlippen 4 gleich. Bereits an dieser Stelle ist darauf hinzuweisen, dass selbstverständlich auch weniger oder mehr als die gezeigten vier Dichtlippen vorgesehen sein können. Im einfachsten Fall ist nur eine Dichtlippe vorgesehen.

Wie Fig. 3 zeigt, weist die respektive jede Dichtlippe 4 eine trapezförmige Querschnittsform auf, mit zwei ebenen Seitenflächen 7, die radial nach außen aufeinander zulaufen. Radial außen liegend ist eine ebene Außenfläche 8 vorgesehen, so dass sich die Trapezform ergibt.

Jede Seitenflanke oder Seitenfläche 7 steht unter einem Winkel zur Oberfläche 9 des Liners 1, wobei in Fig. 3 die beiden Winkel mit *α* und *β* gekennzeichnet sind.

Die beiden Winkel können gleich sein, das heißt, dass *α* = *β* gilt, sie können aber auch ungleich sein, das heißt, dass *α* ≠ *β* ist. In jedem Fall gilt jedoch, dass *α* und *β* stets ungleich 90° sind. Das heißt, dass keine der Seitenflächen 7 senkrecht auf der Oberfläche 9 steht. Im gezeigten Beispiel beträgt jeder der beiden Winkel ca. 105°, wobei dieser Wert nur beispielhaft und variabel ist.

Der erfindungsgemäße Liner zeichnet sich nun durch eine besondere umfangsmäßige Dichtlippengeometrie aus. Denn erfindungsgemäß ist vorgesehen, dass die Höhe der Dichtlippe, also das Maß, wie weit sich die Dichtlippe radial nach außen erstreckt, um den Umfang variiert, also nicht, wie im Stand der Technik der Fall, um den Umfang konstant ist. Die Figuren 4 - 6 zeigen verschiedene Dichtlippenformen im Schnitt entsprechend der Schnittlinien IV, V und VI aus Fig. 1.

Mit dem Pfeil P ist in jeder Figur die Vorderseite des Liners 1 angezeigt, also die frontale Richtung. Der Liner ist so zu tragen, dass dieser frontale Bereich auch vorderseitig am Stumpf zu liegen kommt, im Falle des Unterschenkelstumpfes also vor der Tibia- oder Schienbeinkante.

Gemäß dem Ausführungsbeispiel in Fig. 1 variiert die Höhe der Dichtlippe 4 ausgehend von einem Maximalwert an einer ersten Umfangsposition kontinuierlich zu einem an einer zweiten Umfangsposition gegebenen Minimalwert. In Fig. 1 ist der erste, hier maximale Höhenwert h₁ an einer frontalen Umfangsposition U₁ gegeben und dargestellt, während der zweite, hier minimale Höhenwert h₂ an einer ausgezeichneten zweiten Umfangsposition U₂ gegeben ist, die im gezeigten Ausführungsbeispiel um 180° gegenüberliegend zur ersten Umfangsposition U₁ gegeben ist. Beidseits der ersten Umfangsposition U₁ nimmt die Dichtlippenhöhe kontinuierlich ab, bis sie an der Umfangsposition U₂ den minimalen, zweiten Höhenwert h₂ aufweist. Beispielsweise beträgt der Höhenwert h₁ 6 mm, während der Höhenwert h₂ 4 mm beträgt. In der Tragstellung, wenn also der Liner 1 am Unterschenkelstumpf angeordnet ist, befindet sich die Umfangsposition U₁ im Bereich der respektive vor der Tibia- oder Schienbeinkante, während die Umfangsposition U₂ im Wadenbereich positioniert ist. Das heißt, dass folglich der Bereich mit höher ausgeführter Dichtlippe im frontalen Bereich positioniert ist. Wird nun der mit dem Liner 1 versehene Unterschenkelstumpf in den Prothesenschaft eingeführt, so gelangen die Dichtlippen 4 in Anlage an die Innenwandung des Prothesenschaftes und werden deformiert, so dass sie sich dicht an die Innenwandung anlegen und es in an sich bekannter Weise zur Erzeugung einer hinreichenden Fixierung des Prothesenschaftes kommt. Ein Herausziehen wird durch den sich hierbei ergebenden Unterdruck im Bereich distal der Dichtlippen 4 verhindert.

Ist der Unterschenkelstumpf relativ schlank, so zeigt er im frontalen Bereich eine deutlich von einer Kreisbogenform abweichenden Geometrie auf, resultierend aus dem Umstand, dass bei solchen schlankeren Unterschenkelstümpfen die Querschnittsgeometrie verstärkt durch die knöchernen Strukturen, hier insbesondere das Schienbein, definiert wird. Im frontalen Bereich ist eher eine leicht dreieckförmige Geometrie oder eine medial der Tibia liegende Abflachung gegeben. Dies führt natürlich auch zu einer entsprechenden Geometrie des darüber gezogenen Liners 1.

Während es bei bisher bekannten Linern in solchen Fällen in bestimmten Bewegungssituationen und insbesondere im Sitzen dazu kam, dass sich aufgrund des Verkippens des Stumpfes im Prothesenschaft mitunter die Dichtanlage der Dichtlippe(n) im frontalen Bereich von der Innenwandung des Prothesenschaftes löst und es damit zu einem Verlust des Unterdrucks kommt, ist dies bei dem erfindungsgemäßen Liner 1 ausgeschlossen. Denn aufgrund der größeren Dichtlippenhöhe im frontalen Bereich wird ein etwaiges Verkippen des Stumpfes im Prothesenschaft ausgeglichen. Selbst wenn der Prothesenschaft geringfügig nach hinten kippt, wie dies beim Sitzen oft der Fall ist, bleibt die oder bleiben die Dichtlippen 4 im frontalen Bereich aufgrund ihrer größeren Höhe nach wie vor in ihrer Dichtanlage am Prothesenschaft.

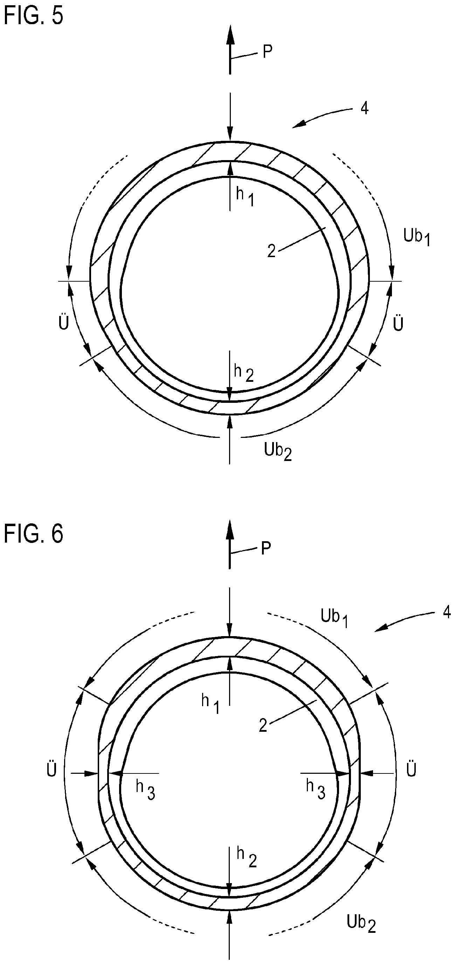

Fig. 5 zeigt ein weiteres Ausführungsbeispiel einer Dichtlippengeometrie in einer Schnittdarstellung. Bei dieser Erfindungsausgestaltung sind zwei sich um den Umfang erstreckende Umfangsbereiche Ub₁ und Ub₂ ausgebildet, innerhalb welcher die Dichtlippe jeweils einen definierten Höhenwert h₁ und h₂ aufweist, das heißt dass die Dichtlippenhöhe in diesen Umfangsbereichen Ub₁ und Ub₂ jeweils konstant ist. Zwischen den beiden Umfangsbereichen Ub₁ und Ub₂ ist jeweils ein Übergangsbereich Ü gegeben, in dem der Höhenwert von h₁ zu h₂ kontinuierlich abnimmt.

Im gezeigten Ausführungsbeispiel befindet sich der Umfangsbereich Ub₁ wiederum im frontalen Linerbereich, er erstreckt sich exemplarisch um ca. 180°, wobei sich dieser Umfangsbereich Ub₁ auch um ein etwas größeres oder kleineres Winkelsegment erstrecken kann. Innerhalb dieses Bereichs ist der konstante Höhenwert h₁ gegeben. h₁ und h₂ stellen wieder Maximal- und Minimalwerte dar.

Zum dorsalen Linerabschnitt hin schließt sich nun jeweils ein Übergangsbereich Ü an, in dem der Höhenwert von h₁ auf h₂ kontinuierlich abnimmt. Im zweiten, dem ersten Übergangsbereich Ub₁ gegenüberliegenden Umfangsbereich Ub₂ ist sodann der konstante Höhenwert h₂ der Dichtlippe 4 gegeben. Dieser zweite Umfangsbereich Ub₂ erstreckt sich im gezeigten Beispiel um ein kleineres Winkelsegment als der erste Umfangsbereich Ub₁, wobei die Ausgestaltung aber auch umgekehrt getroffen sein kann, wie beide Umfangsbereiche Ub₁ und Ub₂ um jeweils ein gleiches Winkelsegment umlaufen können. Wie die Länge der Umfangsbereiche Ub₁ und Ub₂ variabel ist, so ist zwangsläufig auch die Länge der beiden Übergangsbereiche Ü variabel, sie können gleich oder unterschiedlich lang sein.

Von der Funktion her ergibt sich auch hier, dass aufgrund der größeren Dichtlippenhöhe H₁ im frontalen Bereich bei einem etwaigen Verkippen des Stumpfes im Prothesenschaft eine dauerhafte Anlage der oder aller Dichtlippen 4 an der Prothesenschaftinnenwandung sichergestellt ist.

Fig. 6 zeigt schließlich eine weitere Ausführungsform einer Dichtlippengeometrie in einer Schnittdarstellung. Vergleichbar mit der Ausgestaltung gemäß Fig. 5 sind auch hier zwei sich um entsprechende Winkelabschnitte erstreckende

Umfangsbereiche Ub₁ und Ub₂ gegeben, die einander gegenüberliegend positioniert sind. Wiederum ist der Umfangsbereich Ub₁ frontal und der Umfangsbereich Ub₂ dorsal angeordnet. Im Umfangsbereich Ub₁ ist ein konstanter Höhenwert h₁ gegeben, der wie auch bei den vorhergehenden Ausführungsbeispielen beispielsweise 6 mm beträgt, während im Umfangsbereich Ub₂ ein konstanter Höhenwert h₂, der beispielsweise 4 mm beträgt, gegeben ist.

Zwischen den beiden Umfangsbereichen Ub₁ und Ub₂ ist beidseits, also lateral, jeweils auch hier ein Übergangsbereich Ü vorgesehen. Anders als bei der Ausgestaltung gemäß Fig. 5, bei der im Übergangsbereich Ü die Dichtlippenhöhe kontinuierlich von h₁ auf h₂ abnimmt, ist im jeweiligen Übergangsbereich Ü gemäß Fig. 6 die Dichtlippenhöhe noch stärker reduziert. Der in den Übergangsbereichen Ü gegebene Höhenwert h₃ ist kleiner als der im zweiten Umfangsbereich Ub₂ gegebene Höhenwert h₂. Eine solche laterale Höhenreduzierung ist an anatomisch sinnvollen Positionen wie beispielsweise den Seiten denkbar, nachdem üblicherweise ein Verkippen des Unterschenkelstumpfes in dem Prothesenschaft zur Seite hin in geringerem Maß vorkommt und eine etwas geringere Dichtlippenhöhe in diesen Bereich ausreichend ist. Ist aus welchen Gründen auch immer an anatomisch sinnvollen Positionen eine Dichtlippenerhöhen erforderlich sein, so wäre auch dies bei der in Fig. 6 beschriebenen Ausgestaltung ohne weiteres im Übergangsbereich Ü möglich.

Wie bereits den Figuren 4 - 6 dem Grunde nach zu entnehmen ist, besteht beim erfindungsgemäßen Liner 1 auch die Möglichkeit, die Wanddicke des Linerkörpers 2 im Dichtlippenabschnitt 3 zu variieren. Da eine zu hohe Linerkörperwandstärke im Bereich der Kniekehle vom Träger beim Beugen des Knies aufgrund der zustande kommenden Faltenbildung als unangenehm empfunden wird, wird der Linerkörper 2 möglichst dünnwandig ausgelegt. Andererseits besteht insbesondere bei schlanken Unterschenkelstümpfen mit dominanter Tibiakante das Problem, dass Bereiche geringer Weichteildeckung gegeben sind, an denen es im Dichtlippenbereich bei zu geringer Linerkörperwandstärke zu einer Druckbelastung des Stumpfes kommen kann, was das Entstauen des Gliedmaßenstumpfes erschweren oder Läsionen zur Folge haben kann. Daher variiert neben der Dichtlippenhöhe auch die Wandstärke des Linerkörpers 2 im Dichtlippenabschnitt 3, wie dies bereits in den Figuren 4 - 6 dargestellt ist.

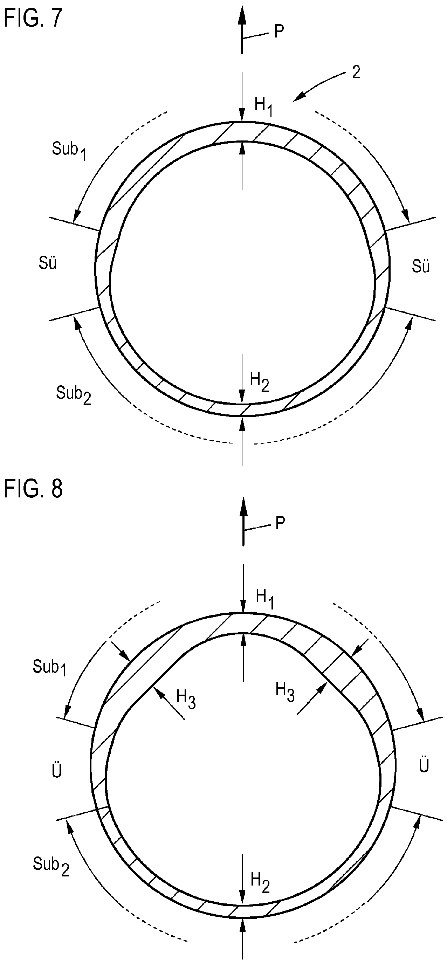

Fig. 7 zeigt eine vergrößerte Schnittansicht des Linerkörpers 2 entlang der Linie VII aus Fig. 1. Angegeben ist wiederum die Frontalrichtung P. Ersichtlich weist der Linerkörper 2 einen ersten Linerkörperumfangsbereich Sub₁ auf, der frontal ausgebildet ist, in dem eine erste Wandstärke H₁ des Linerkörpers 2 gegeben ist. Des Weiteren ist ein zweiter Linerkörperumfangsbereich Sub₂ gegeben, der eine reduzierte Wandstärke H₂ aufweist, wobei zwischen beiden ein Linerkörperübergangsbereich Sü gegeben ist, in dem die Linerkörperwandstärke vom Wert H₁ auf den Wert H₂ abnimmt. Dadurch, dass die Linerkörperwandstärke im frontalen Bereich, also in dem Bereich, in dem das Schienbein umgriffen ist, stärker ausgelegt ist, ist folglich dieser druckempfindliche Bereich stärker unterfüttert, mithin also der druckempfindliche Schienbeinbereich über den dämpfenden Linerkörper 2 besser geschützt.

Die Variation der Linerkörperwandstärke ist wie beschrieben bevorzugt nur im Dichtlippenbereich 3 vorgesehen. Insbesondere oberhalb respektive proximal des Dichtlippenbereichs 3 ist der Linerkörper 2 mit einer konstanten Wandstärke, beispielsweise der Wandstärke H₂, ausgeführt, das heißt, dass die Wandstärke wieder auf einen konstanten, geringen Wert abnimmt, damit sich beispielsweise über dem Knie, wiederum in einem frontalen Bereich, nicht zu viel Linerkörpermaterial befindet, was das Abbeugen des Knies unnötig erschweren würde, bzw. im Bereich der Kniekehle, also im dorsalen Bereich relativ wenig Material befindet und es nur in geringem Maß zu einem Faltenwurf kommt. Zum distalen Linerende hin kann die Wandstärkenvariation aus dem Dichtlippenabschnitt beibehalten werden, alternativ kann auch hier eine umfänglich gleichmäßige dünne Wandstärke vorgesehen sein.

Fig. 8 zeigt eine weitere Schnittansicht durch den Linerkörper 2 entlang der Linie VIII - VIII und eine Darstellung einer weiteren Querschnittsgeometrie des Linerkörpers. Auch hier variiert ersichtlich die Wandstärke des Linerkörpers 2 beachtlich. In einem ersten Linerkörperumfangsbereich Sub₁ ist an einer frontalen Position wiederum ein erster Wandstärkenwert H₁ gegeben, der bereits größer ist als der im zweiten Linerkörperumfangsbereich Sub₂, in dem der niedrigere Wandstärkenwert H₂ gegeben ist. Um sich jedoch im frontalen Bereich noch besser der mitunter stark von einer rundlichen Form abweichenden Unterschenkelgeometrie anpassen zu können, vergrößert sich die Wandstärke im Linerkörperumfangsbereich Sub₁ ausgehend vom Wert H₁ deutlich auf einen Wandstärkenwert H₃, wobei hierzu die Linerkörperwandung nach innen hin aufgedickt wird, so dass sich die in Fig. 8 gezeigte spezifische Geometrie ergibt. Diese Aufdickung erfolgt durch einen kontinuierlichen Dickenzuwachs und zu den beiden seitlichen Enden des Linerkörperumfangsbereichs Sub₁ hin durch eine entsprechende kontinuierliche Abnahme und einen Übergang in den jeweiligen lateralen Übergangsbereich Ü, in dem die Wandstärke kontinuierlich abnimmt, bis der Wandstärkenwert H₂ erreicht ist. Das heißt, dass bei dieser Ausgestaltung des erfindungsgemäßen Liners die Linerkörperwandstärke links und rechts der Tibiakante erhöht ist, mithin also radial nach innen hin aufgedickt ist. Alternativ ist es auch denkbar, dass die in Figur 8 beschriebene Dickenerhöhung auf den Wert H₃ nur einseitig der Tibikante erfolgt.

## Patentansprüche

1. Liner zum Überziehen über einen Gliedmaßenstumpf zur Fixierung eines Prothesenschafts, mit einem hohlen Linerkörper und wenigstens einer umlaufenden, seitlich vom Linerkörper abstehenden flexiblen Dichtlippe zur dichten Anlage an der Innenseite des Prothesenschafts, **dadurch gekennzeichnet, dass** die Höhe der Dichtlippe (4) um ihren Umfang von einem ersten Höhenwert (h₁) an einer ersten Umfangsposition (U₁) oder in einem ersten Umfangsbereich (Ub₁) zu einem niedrigeren zweiten Höhenwert (h₂) an einer zweiten Umfangsposition (U₂) oder in einem zweiten Umfangsbereich (Ub₂) variiert.

2. Liner nach Anspruch 1, **dadurch gekennzeichnet, dass** die erste Umfangsposition (U₁) oder der erste Umfangsbereich (Ub₁) und die zweite Umfangsposition (U₂) oder der zweite Umfangsbereich (Ub₂) einander gegenüber liegen.

3. Liner nach Anspruch 1 oder 2, **dadurch gekennzeichnet, dass** mehrere Dichtlippen (4) vorgesehen sind, wobei die Höhe nur einer, mehrerer oder aller Dichtlippen (4) variiert.

4. Liner nach einem der vorangehenden Ansprüche, **dadurch gekennzeichnet, dass** die Höhe ausgehend von einer den ersten Höhenwert (h₁) aufweisenden ersten Umfangsposition (U₁) zu dem an einer zweiten Umfangsposition (U₂) gegebenen zweiten Höhenwert (h₂) abnimmt.

5. Liner nach einem der Ansprüche 1 bis 3, **dadurch gekennzeichnet, dass** die Höhe in einem Umfangsbereich (Ub₁) den ersten Höhenwert (h₁) aufweist und anschließend zu dem zweiten Höhenwert (h₂) abnimmt.

6. Liner nach einem der Ansprüche 1 bis 3, **dadurch gekennzeichnet, dass** die Höhe in einem ersten Umfangsbereich (Ub₁) den ersten Höhenwert (h₁) aufweist, dass die Höhe in einem daran anschließenden Übergangsbereich (Ü) abnimmt, und dass die Höhe in dem daran anschließenden zweiten Umfangsbereich (Ub₂) den zweiten Höhenwert (h₂) aufweist.

7. Liner nach Anspruch 6, **dadurch gekennzeichnet, dass** die Höhe im Übergangsbereich (Ü) abschnittsweise kleiner ist als der im zweiten Umfangsbereich (Ub₂) gegebene zweite Höhenwert (h₂) oder größer als der im ersten Umfangsbereich (Ub₁) gegebene erste Höhenwert (h₂).

8. Liner nach einem der Ansprüche 1 bis 3, **dadurch gekennzeichnet, dass** die Höhe ausgehend von einer den ersten Höhenwert (h₁) aufweisenden Umfangsposition (U₁) zu dem zweiten Höhenwert (h₂) abnimmt, der in einem Umfangsbereich (Ub₂) gegeben ist.

9. Liner nach einem der vorangehenden Ansprüche, **dadurch gekennzeichnet, dass** der erste Höhenwert (h₁) zwischen 4 - 8 mm, insbesondere 6 mm und der zweite Höhenwert (h₂) zwischen 2 - 5 mm, insbesondere 4 mm beträgt.

10. Liner nach einem der vorangehenden Ansprüche, **dadurch gekennzeichnet, dass** die Dichtlippe (4) eine trapezförmige Querschnittsform aufweist, mit zwei ebenen Seitenflächen (7) und einer diese verbindenden ebenen Außenfläche (8).

11. Liner nach Anspruch 10, **dadurch gekennzeichnet, dass** der Winkel (α, β), den die beiden Seitenflächen (7) zur Oberfläche (9) des Liners (1) einnehmen, gleich oder unterschiedlich ist, jedoch stets ungleich 90° ist.

12. Liner nach einem der vorangehenden Ansprüche, **dadurch gekennzeichnet, dass** die Wandstärke des Linerkörpers (2) im Bereich der einen oder der mehreren Dichtlippen (4) um den Umfang variiert, wobei die Wandstärke in dem Bereich, in dem die eine oder die mehreren Dichtlippe dem ersten Höhenwert (h₁) aufweisen, größer ist als in dem Bereich, in dem die eine oder die mehreren Dichtlippen den zweiten Höhenwert (h₂) aufweisen.

13. Liner nach Anspruch 12, **dadurch gekennzeichnet, dass** die Wandstärke in einer ersten Linerkörperumfangsposition oder in einem ersten Linerkörperumfangsbereich (Sub₁), die oder der der Umfangsposition (U₁) oder dem Umfangsbereich (Ub₁) mit der den ersten Höhenwert aufweisenden Dichtlippenhöhe zugeordnet ist, größer ist als in einer zweiten Linerkörperumfangsposition oder in einem zweiten Linerkörperumfangsbereich (Sub₂), die oder der der Umfangsposition (U₂) oder dem Umfangsbereich (Ub₂) mit der den zweiten Höhenwert aufweisenden Dichtlippenhöhe zugeordnet ist.

14. Liner nach Anspruch 13, **dadurch gekennzeichnet, dass** die Wandstärke zumindest in einem Bereich zwischen der ersten und der zweiten Linerkörperumfangsposition oder dem ersten und dem zweiten Linerkörperumfangsbereich (Sub₁, Sub₂) abnimmt, oder dass die Wandstärke zumindest in einem Bereich zwischen der ersten Linerkörperumfangsposition und der zweiten Linerkörperumfangsposition oder dem ersten und dem zweiten Linerkörperumfangsbereich (Sub₁, Sub₂) ausgehend von der ersten Linerkörperumfangsposition oder vom ersten Linerkörperumfangsbereich (Sub₁) zunimmt und zur zweiten Linerkörperumfangsposition oder zum zweiten Linerkörperumfangsbereich (Sub₂) hin abnimmt.

15. Liner nach einem der Ansprüche 12 bis 14, **dadurch gekennzeichnet, dass** die Wandstärke des Linerkörpers (2) proximal der einen oder der mehreren Dichtlippen (4) abnimmt.

16. Liner nach einem der vorangehenden Ansprüche, **dadurch gekennzeichnet, dass** der Linerkörper (2) in dem Bereich proximal der einen oder der mehreren Dichtlippen (4) mit einem, vorzugsweise sowohl in axialer als auch radialer Richtung dehnbaren, textilen Verstärkungsmaterial (6) versehen ist.

## Claims

1. A liner for pulling over a limb stump for the fixation of a prosthesis shaft, having a hollow liner body and at least one circumferential, flexible sealing lip that projects laterally from the liner body for sealing engagement with the inner side of the prosthesis shaft, **characterized in that** the height of the sealing lip (4) around its circumference varies from a first height value (h₁) at a first circumferential position (U₁) or in a first circumferential region (Ub₁) to a lower second height value (h₂) at a second circumferential position (U₂) or in a second circumferential region (Ub₂).

2. The liner according to claim 1, **characterized in that** the first circumferential position (U₁) or the first circumferential region (Ub₁) and the second circumferential position (U₂) or the second circumferential region (Ub₂) are disposed opposite to one another.

3. The liner according to claim 1 or 2, **characterized in that** several sealing lips (4) are provided, wherein the height of only one, several or all sealing lips (4) varies.

4. The liner according to any of the preceding claims, **characterized in that** the height decreases starting from a first circumferential position (U₁) having the first height value (h₁) to the second height value (h₂) given at a second circumferential position (U₂).

5. The liner according to any of claims 1 to 3, **characterized in that** the height in a circumferential region (Ub₁) has the first height value (h₁) and subsequently decreases to the second height value (h₂).

6. The liner according to any of claims 1 to 3, **characterized in that** the height in a first circumferential region (Ub₁) has the first height value (h₁), that the height in an adjoining transition region (U) decreases, and that the height in the second circumferential region (Ub₂) adjoining thereto has the second height value (h₂).

7. The liner according to claim 6, **characterized in that** the height in the transition region (U) in sections is smaller than the second height value (h₂) given in the second circumferential region (Ub₂), or greater than the first height value (h₁) given in the first circumferential region (Ub₁).

8. The liner according to any of claims 1 to 3, **characterized in that** the height decreases starting from a circumferential position (U₁) having the first height value (h₁) to the second height value (h₂) given in a circumferential region (Ub₂).

9. The liner according to any of the preceding claims, **characterized in that** the first height value (h₁) amounts to between 4-8 mm, in particular 6 mm, and the second height value (h₂) amounts to between 2-5 mm, in particular 4 mm.

10. The liner according to any of the preceding claims, **characterized in that** the sealing lip (4) has a trapezoidal cross-sectional shape, with two flat side faces (7) and a flat outer face (8) connecting these.

11. The liner according to claim 10, **characterized in that** the angle (α, β) occupied by the two side faces (7) to the surface (9) of the liner (1) is the same or different, but is always unequal to 90°.

12. The liner according to any of the preceding claims, **characterized in that** the wall thickness of the liner body (2) in the region of the one or several sealing lips (4) varies around the circumference, wherein the wall thickness in the region in which the one or the several sealing lip(s) have the first height value (h₁) is greater than in the region in which the one or the several sealing lips have the second height value (h₂).

13. The liner according to claim 12, **characterized in that** the wall thickness in a first liner body circumferential position or in a first liner body circumferential region (Sub₁) which is allocated to the circumferential position (U₁) or to the circumferential region (Ub₁) with the sealing lip height having the first height value is greater than in a second liner body circumferential position or in a second liner body circumferential region (Sub₂) which is allocated to the circumferential position (U₂) or to the circumferential region (Ub₂) with the sealing lip height having the second height value.

14. The liner according to claim 13, **characterized in that** the wall thickness decreases, at least in a region between the first and the second liner body circumferential position or the first and the second liner body circumferential region (Sub₁, Sub₂), or that the wall thickness increases at least in a region between the first liner body circumferential position and the second liner body circumferential position or the first and the second liner body circumferential region (Sub₁, Sub₂) starting from the first liner body circumferential position or from the first liner body circumferential region (Sub₁), and decreases towards the second liner body circumferential position or the second liner body circumferential region (Sub₂).

15. The liner according to any of claims 12 to 14, **characterized in that** the wall thickness of the liner body (2) decreases proximally to the one or the several sealing lips (4).

16. The liner according to any of the preceding claims, **characterized in that** the liner body (2) is equipped in the region proximal to the one or the several sealing lips (4) with a textile reinforcing material (6) that is preferably stretchable both in the axial and radial directions.

## Revendications

1. Manchon destiné à être enfilé sur un moignon de membre pour la fixation d'une tige prothétique, comprenant un corps de manchon creux et au moins une lèvre d'étanchéité flexible, agencée sur tout le pourtour en dépassant latéralement du corps de manchon et destinée à une jointure étanche à la face intérieure de la tige prothétique, **caractérisé en ce que** la hauteur de la lèvre d'étanchéité (4) varie, sur son pourtour, entre une première valeur de hauteur (h₁) à une première position de pourtour (U₁) ou dans une première zone de pourtour (Ub₁), et une deuxième valeur de hauteur (h₂), plus faible, à une deuxième position de pourtour (U₂) ou dans une deuxième zone de pourtour (Ub₂).

2. Manchon selon la revendication 1, **caractérisé en ce que** la première position de pourtour (U₁) ou la première zone de pourtour (Ub₁) et la deuxième position de pourtour (U₂) ou la deuxième zone de pourtour (Ub₂) se font face.

3. Manchon selon la revendication 1 ou 2, **caractérisé en ce que** plusieurs lèvres d'étanchéité (4) sont prévues, cependant que la la hauteur de seulement une, de plusieurs ou de toutes les lèvres d'étanchéité (4) varie.

4. Manchon selon une des revendications précédentes, **caractérisé en ce que** la hauteur diminue à partir d'une première position de pourtour (U₁) présentant la première valeur de hauteur (h₁) jusqu'à à la deuxième valeur de hauteur (h₂) donnée à une deuxième position de pourtour (U₂)

5. Manchon selon une des revendications de 1 à 3, **caractérisé en ce que** la hauteur présente la première valeur de hauteur (h₁) dans une zone de pourtour (Ub₁), puis diminue vers la deuxième valeur de hauteur (h₂).

6. Manchon selon une des revendications de 1 à 3, **caractérisé en ce que** la hauteur présente la première valeur de hauteur (h₁) dans une première zone de pourtour (Ub₁), **en ce que** la hauteur diminue dans une zone de transition (Ü) y étant adjacente, et **en ce que** la hauteur présente la deuxième valeur de hauteur (h₂) dans la deuxième zone de pourtour (Ub₂) y étant adjacente.

7. Manchon selon la revendication 6, **caractérisé en ce que** la hauteur est, dans la zone de transition (Ü), par tronçons inférieure à la deuxième valeur de hauteur (h₂) donnée dans la deuxième zone de pourtour (Ub₂) ou supérieure à la première valeur de hauteur (h₂) donnée dans la première zone de pourtour (Ub₁).

8. Manchon selon une des revendications de 1 à 3, **caractérisé en ce que** la hauteur diminue à partir d'une première position de pourtour (U₁) présentant la première valeur de hauteur (h₁) jusqu'à la deuxième valeur de hauteur (h₂) donnée dans une zone de pourtour (Ub₂).

9. Manchon selon une des revendications précédentes, **caractérisé en ce que** la première valeur de hauteur (h₁) est comprise entre 4 et 8 mm, s'élevant en particulier à 6 mm, et que la deuxième valeur de hauteur (h₂) est comprise entre 2 et 5 mm, s'élevant en particulier à 4 mm.

10. Manchon selon une des revendications précédentes, **caractérisé en ce que** la lèvre d'étanchéité (4) a une forme en section transversale trapézoïdale ayant deux faces latérales (7) planes et une face extérieure (8) plane reliant ces dernières.

11. Manchon selon la revendication 10, **caractérisé en ce que** l'angle (α, β) que les deux faces latérales (7) planes forment avec la surface (9) du manchon (1) est identique ou différent, mais néanmoins toujours inégal à 90°.

12. Manchon selon une des revendications précédentes, **caractérisé en ce que** l'épaisseur de paroi du corps de manchon (2) varie sur le pourtour dans la zone de la une ou des plusieurs lèvres d'étanchéité (4), l'épaisseur de paroi étant plus élevée dans la zone dans laquelle la une ou les plusieurs lèvres d'étanchéité présentent la première valeur de hauteur (h₁) que dans la zone dans laquelle la une ou les plusieurs lèvres d'étanchéité présentent la deuxième valeur de hauteur (h₂).

13. Manchon selon la revendication 12, **caractérisé en ce que** l'épaisseur de paroi est, dans une première position de pourtour du corps de manchon ou dans une première zone de pourtour du corps de manchon (Sub₁) associée à la position de pourtour (U₁) ou à la zone de pourtour (Ub₁) dont la hauteur de lèvre d'étanchéité présente la première valeur de hauteur, plus grande que dans une deuxième position de pourtour du corps de manchon ou dans une deuxième zone de pourtour du corps de manchon (Sub₂) associée à la position de pourtour (U₂) ou à la zone de pourtour (Ub₂) dont la hauteur de lèvre d'étanchéité présente la deuxième valeur de hauteur.

14. Manchon selon la revendication 13, **caractérisé en ce que** l'épaisseur de paroi diminue au moins dans une zone située entre la première et la deuxième position de pourtour du corps de manchon ou entre la première et la deuxième zone de pourtour du corps de manchon (Sub₁, Sub₂), ou **en ce que** l'épaisseur de paroi augmente au moins dans une zone située entre la première position de pourtour du corps de manchon et la deuxième position de pourtour du corps de manchon ou entre la première et la deuxième zone de pourtour du corps de manchon (Sub₁, Sub₂) à partir de la première position de pourtour du corps de manchon ou de la première zone de pourtour du corps de manchon (Sub₁), et diminue vers la deuxième position de pourtour du corps de manchon ou vers la deuxième zone de pourtour du corps de manchon (Sub₂).

15. Manchon selon une des revendications de 12 à 14, **caractérisé en ce que** l'épaisseur de paroi du corps de manchon (2) diminue à proximité de la une ou des plusieurs lèvres d'étanchéité (4).

16. Manchon selon une des revendications précédentes, **caractérisé en ce que** le corps de manchon (2) est, dans la zone située à proximité de la une ou des plusieurs lèvres d'étanchéité (4), pourvu, de préférence tant dans le sens axial que dans le sens radial, d'une matière textile (6) élastique.
